# EUROPÄISCHE PATENTANMELDUNG

(11) **EP 3 574 933 A1**
(43) Veröffentlichungstag der Anmeldung: **04.12.2019**
(21) Anmeldenummer: 18174693.4
(22) Anmeldetag: 29.05.2018
(51) Int. Cl.: A61M 1/10, A61M 1/12

(54) **BLUTPUMPE MIT EINEM ANTREIBBAREN ROTOR UND ZWEI LAGERN**

(71) Anmelder: Berlin Heart GmbH, 12247 Berlin (DE)
(72) Erfinder: Richert, Dr. Hendryk, 12487 Berlin (DE); Peters, Oliver, 14129 Berlin (DE)
(74) Vertreter: Pfenning, Meinig & Partner mbB

(57) **Zusammenfassung**

Die Erfindung bezieht sich auf eine Blutpumpe mit einem antreibbaren Rotor (1), der ein oder mehrere Förderelemente (2) zur Förderung von Blut aufweist und der an seinem ersten Ende (3a) in einem ersten Lager (4) und an seinem zweiten Ende (3b) in einem zweiten Lager (5) drehbar gelagert ist, wobei die Form und Rotationsrichtung des Rotors derart eingerichtet sind, dass im Betrieb eine Rückwirkungskraft des geförderten Blutes den Rotor in Axialrichtung (6) gegen das erste Lager (4) drückt, wobei eine Kompressionseinrichtung vorgesehen ist, die eine axiale Kompressionskraft zwischen einem mit dem Rotor rotierenden Lagerelement (5a) des zweiten Lagers und einem mit dem Pumpengehäuse (7) verbundenen Element (5b) des zweiten Lagers (5) erzeugt und dabei möglichst geringe Kraftänderungen im ersten Lager generiert.

## Beschreibung

Die Erfindung liegt auf dem Gebiet des Maschinenbaus und ist mit besonderem Vorteil auf dem Gebiet der Pumpentechnik einsetzbar. Speziell ist die Erfindung auf dem medizintechnischen Gebiet bei der Konstruktion von Blutpumpen verwendbar.

Blutpumpen sind in den vergangenen Jahren stetig weiterentwickelt worden. So sind bereits diverse implantierbare Pumpen bekannt, sowie Pumpen, die außen am Körper des Patienten getragen werden. Derartige Pumpen weisen in vielen Ausführungsformen Rotoren auf, die Förderelemente, beispielsweise in der Form von Schaufeln oder schraubenähnlichen Förderflächen, aufweisen, durch die bei Rotation des Rotors Blut gefördert werden kann.

Ein bekanntes Problem stellt dabei die mechanische Lagerung von Rotoren dar, da von den Blutpumpen eine lange Standzeit und hohe Zuverlässigkeit gefordert wird. Zudem liegt bei der Lagerung ein Problem darin, dass die Lagerstellen in vielen Fällen als sphärische oder kegelförmige Lager ausgelegt sind, die teilweise vom Blut durchströmt werden oder mit dem zu fördernden Blut in Kontakt kommen. Dabei sind die Lager oft so ausgestaltet, dass die axiale Fesselung des Rotors konstruktionsbedingt einen kumulierten Axialspalt im Bereich von 1 bis einigen 100 Mikrometern erfordert. Ist dieser axiale Spalt zu klein, erhöht sich u. U. die Reibleistung im Lager, und es besteht die Gefahr einer Thrombenbildung aufgrund der durch die erhöhte Reibung erhöhten Lagertemperatur. Außerdem können aus den Lagern herausgelöste, harte Mikropartikel, die im Durchmesser größer als der eingestellte Lagerspalt sind, den Spalt nicht verlassen und zu erhöhter Reibleistung mit einhergehender Temperaturerhöhung im Lager führen. Die Thromben bleiben dann unter Umständen am Lager oder in der Pumpe haften oder werden als Mikrothromben in den Körper gespült und können dort zu Komplikationen wie Gefäßverstopfungen führen, die im schlimmsten Fall Schlaganfälle zur Folge haben. Ist der Lagerspalt hingegen zu groß, wird Blut durch diesen Spalt gefördert, wobei aufgrund dort vorherrschender hoher Scherung die Blutbestandteile Schaden nehmen (Hämolyse).

Wird der Spalt noch weiter vergrößert, wird das Lager instabil, und die Lebensdauer des Lagers und damit der gesamten Blutpumpe sinkt. Als Lagermaterialen kommen hämokompatible, harte Materialien mit guten Gleit- und Wärmeleiteigenschaften zum Einsatz wie zum Beispiel, aber nicht ausschließlich, Saphir, Rubin, SiC, A₂O₃, Si₃N₄, Diamant oder hämokompatible Hartmetalllegierungen. Darüber hinaus sind auch Beschichtungen der Lager zur Verbesserung der Gleiteigenschaften oder der Hämokompatiblilität, wie DLC, Parylene, MPC, TIOx usw., denkbar. Somit wird deutlich, dass die Axialspaltkontrolle eine wesentliche Herausforderung bei der Nutzung mechanischer Lager in Blutpumpen darstellt.

Bisher wird versucht, dem genannten Effekt der Blutschädigung dadurch entgegenzuwirken, dass der festeingestellte axiale Spalt im mechanischen Lager so weit minimiert wird, dass der Rotor mit gerade noch tolerierbarer Reibleistung läuft. Da die Lager über viele Jahre im Patientenkörper verbleiben, muss der Verschleiß der Komponenten, die mit Blut in Kontakt kommen, durch Einsatz widerstandsfähiger Materialien minimiert werden. Beispielsweise kann der Materialverlust an einem solchen Lager bei einer Betriebsdauer von einem Jahr in der Größenordnung von wenigen Mikrometern gehalten werden. Allerdings wird der Axialspalt im Lager um genau diesen Betrag von Jahr zu Jahr steigen, so dass mit zunehmender Betriebsdauer auch die Hämolysewerte der Pumpen ansteigen werden. Davon abgesehen stellen freigesetzte Mikropartikel eine dauerhafte Gefahr dar.

Der vorliegenden Erfindung liegt vor dem Hintergrund des Standes der Technik die Aufgabe zugrunde, bei einer Blutpumpe der eingangs genannten Art auf Dauer und auch unter Berücksichtigung von Lagerabrieb ein minimiertes Lagerspiel zu erreichen und dabei sowohl die hämolytischen als auch die thromboembolischen Eigenschaften der Lager zu optimieren.

Die Aufgabe wird mit dem Merkmal der Erfindung gemäß Patentanspruch 1 gelöst. Die Patentansprüche 2 bis 11 zeigen mögliche Implementierungen einer solchen Blutpumpe auf, ohne dabei jedoch auf diese Beispiele beschränkt zu sein. Dem mit der Materie vertrauten Ingenieur werden noch weitere, ähnliche Lagerspaltkontrollmechanismen in den Sinn kommen.

Demgemäß bezieht sich die Erfindung auf eine Blutpumpe mit einem antreibbaren Rotor, der ein oder mehrere Förderelemente zur Förderung von Blut aufweist und der an seinem ersten Ende in einem ersten Lager und an seinem zweiten Ende in einem zweiten Lager drehbar gelagert ist, wobei die Form und Rotationsrichtung des Rotors derart eingerichtet sind, dass im Betrieb eine Rückwirkungskraft des geförderten Blutes den Rotor in Axialrichtung gegen das erste Lager drückt. Der Rotor kann eine Welle aufweisen, wobei das erste Ende des Rotors dann durch das erste Ende der Welle und das zweite Ende des Rotors durch das zweite Ende der Welle gebildet ist und die Wellenenden jeweils im ersten und zweiten Lager gelagert sind.

Die Aufgabe, dauerhaft das Lagerspiel zu minimieren, wird gemäß der Erfindung dadurch gelöst, dass eine Kompressionseinrichtung vorgesehen ist, die eine axiale Kompressionskraft zwischen einem mit dem Rotor rotierenden Lagerelement eines Lagers, insbesondere des zweiten Lagers, und einem mit dem Pumpengehäuse verbundenen Element des Lagers, insbesondere des zweiten Lagers, erzeugt.

Da im Betrieb durch die Rückwirkungskraft des geförderten Blutes ohnehin eine axiale Kompressionskraft auf das erste Lager wirkt, wird durch die genannte Maßnahme und die zusätzliche Kompression des zweiten Lagers in der Axialrichtung insgesamt das Lagerspiel der Rotorwelle vermindert. Entsteht durch den Abrieb im Lager ein Materialschwund, so wird dieser durch die wirkenden Axialkräfte ausgeglichen, so dass das Lagerspiel im ersten und zweiten Lager nahezu unverändert gehalten werden kann. Ein Schlagen des zweiten Lagers nach fortschreitender Abnutzung mit der entsprechend zu befürchtenden Schädigung von Blut kann somit vermieden werden.

Eine vorteilhafte Ausgestaltung der Erfindung kann vorsehen, dass die Kompressionseinrichtung wenigstens zwei Magnetelemente in Form zweier Magnete oder eines Magneten und eines magnetisierbaren Elements, insbesondere eines weichmagnetischen Elements, aufweist. Auf diese Weise kann in kontrollierter Form eine Anziehungskraft oder eine Abstoßungskraft zwischen den beiden Magnetelementen erzeugt und als Kompressionskraft genutzt werden. Die Kraftcharakteristik einer solchen Magnetanordnung erlaubt die Erzeugung von stabilen und konstanten oder wenig veränderlichen Kräften auch über einen Kompressionsweg. Wenn im weiteren Verlauf dieser Beschreibung von Magneten gesprochen wird, so sind damit auch jeweils Magnetanordnungen mit Magnetelementen der oben erläuterten Art gemeint und umfasst.

Die Erfindung kann zudem dadurch ausgestaltet werden, dass eines oder beide der Lagerelemente des zweiten Lagers relativ zu dem Pumpengehäuse und/oder dem Rotor, insbesondere dem ersten Ende des Rotors, axial verschiebbar sind. Dabei können die verschiebbaren Lagerelemente verdrehsicher mit den Bauteilen verbunden sein, an denen sie gehalten/befestigt sind. Zudem kann jeweils ein mechanischer Anschlag für die Axialbewegung des Lagerelements / der Lagerelemente vorgesehen sein.

Eine weitere vorteilhafte Ausgestaltung der Erfindung kann vorsehen, dass die Kompressionseinrichtung ein erstes Magnetelement, insbesondere einen ersten Magneten, aufweist, der mit dem Rotor verbunden ist, sowie ein zweites Magnetelement, insbesondere einen zweiten Magneten, der mit dem Pumpengehäuse verbunden und axial vor oder hinter dem zweiten Ende des Rotors oder der Welle angeordnet ist, wobei zwischen dem ersten und dem zweiten Magneten eine Anziehungskraft oder eine Abstoßungskraft wirkt. Durch diese Ausgestaltung der Erfindung wird die Welle durch die Magnetkraft zu den mit dem Pumpengehäuse verbundenen Lagerteilen des zweiten Lagers gezogen, so dass die erfindungsgemäße axiale Kompressionskraft im zweiten Lager entsteht. Die axiale Andruckkraft der Welle gegen das erste Lager bzw. die mit dem Pumpengehäuse verbundenen Teile des ersten Lagers werden in diesem Fall um die Kompressionskräfte im zweiten Lager vermindert. Daher sollten die Magnetfeldstärken der Magnete im Bereich des zweiten Lagers so groß gewählt werden, dass die Kompressionskraft im zweiten Lager deutlich geringer ist als die durch die Rückwirkungskraft des Blutes im ersten Lager wirkende Kompressionskraft.

Dabei kann die Anordnung von Magneten im Bereich des zweiten Lagers derart gestaltet sein, dass in den feststehenden, mit dem Pumpengehäuse verbundenen Teilen des zweiten Lagers ein Stabmagnet oder Ringmagnet und innerhalb der Welle ebenfalls ein Stabmagnet oder ein in die Welle eingelassener Ringmagnet angeordnet ist. Die Orientierung der Magnete im feststehenden Bereich des Lagers und an der Welle muss so gestaltet sein, dass die Magnete sich gegenseitig anziehen. Es kann auch alternativ dazu im Bereich der feststehenden Lagerteile des zweiten Lagers ein Ringmagnet und im Bereich der Welle ein Stabmagnet oder ein Ringmagnet vorgesehen sein. Der magnetische Kreis zwischen den zwei Magneten im Bereich des zweiten Lagers wird entweder über Streufelder oder über magnetflussleitende Teile in Wellenkörper und Pumpengehäuse geschlossen. Die Form der Magnete oder magnetischen Elemente kann vielfältig sein und beispielsweise die Form von Stäben, Quadern, Ringen oder Kombinationen der genannten Formen haben. Die hier genannten Beispiele sind nicht als einschränkend zu verstehen.

Es kann in einer Ausführungsform der Erfindung vorgesehen sein, dass ein mit dem Pumpengehäuse verbundenes Element des zweiten Lagers am Pumpengehäuse oder ein mit dem Rotor verbundenes Element des zweiten Lagers relativ zu dem ersten Ende des Rotors axial verschiebbar ist. Damit kann das am Pumpengehäuse axial verschiebbare Element des zweiten Lagers bei Materialschwund durch Abnutzung in Richtung zur Welle des Rotors hin verschoben werden.

Zudem kann vorgesehen sein, dass ein mit dem Pumpengehäuse verbundenes Element des ersten oder zweiten Lagers am Pumpengehäuse mittels eines Federelements axial verschiebbar abgestützt ist, wobei das Federelement derart vorgespannt ist, dass es das mit dem Pumpengehäuse verbundene Lagerelement in Axialrichtung gegen das rotierende Lagerelement des ersten oder zweiten Lagers drückt. In diesem Fall muss das Federelement so gestaltet sein, dass die entstehenden Federkräfte relativ gering sind, insbesondere wesentlich geringer als die Axialkraft, die durch die Rückwirkungskraft des Blutes im ersten Lager entsteht, um die auf die Welle wirkenden Kräfte in Richtung des ersten Lagers insgesamt nicht zu groß werden zu lassen.

Es kann dabei vorteilhaft vorgesehen sein, dass das axial verschiebbare, mit dem Pumpengehäuse verbundene Lagerelement des zweiten Lagers wenigstens teilweise in dem blutdurchströmten Raum der Pumpe angeordnet und gegenüber dem Außenraum durch eine fluiddichte Membran abgedichtet ist.

Wenn das mit dem Pumpengehäuse verbundene, axial verschiebbare Lagerelement des zweiten Lagers in einfacher Weise und ohne hohen Kraftaufwand verschiebbar sein soll, dann können sich im Bereich der mechanischen Führung, die für diese Verschiebungsbewegung vorgesehen ist, Spalte ausbilden, die nicht fluiddicht sind. Für diesen Fall ist die am Pumpengehäuse abgedichtete Membran vorgesehen, die den Austritt von Blut aus der Blutpumpe verhindert.

Es kann zudem vorgesehen sein, dass die Membran elastisch ausgebildet ist und das Federelement bildet, das das mit dem Pumpengehäuse verbundene Lagerelement des zweiten Lagers gegen das rotierende Lagerelement des zweiten Lagers drückt. Damit bildet die Membran, die aus einem dünnen Blech, einem Kunststoff oder einem Elastomer gebildet sein kann, das Federelement, das dauerhaft eine elastische Axialkraft auf die mit dem Pumpengehäuse verbundenen Elemente des zweiten Lagers ausübt. Der Weg, den die Membran zum Nachschieben des mit dem Pumpengehäuse verbundenen Lagerelements des zweiten Lagers zurücklegen muss, beschränkt sich auf wenige Mikrometer, so dass die Membran auch aus Stoffen bestehen kann, die nur wenig verformbar sind. Jedoch erlaubt die Herstellung der Membran aus einem Elastomer die Implementierung geringer Federkonstanten und damit eine gleichbleibende Kraft auch bei einem zurückgelegten Federweg oder der notwendigen axialen Verschiebung von Lagerelementen.

Es kann in einer alternativen Implementierung der Erfindung auch vorgesehen sein, dass der Rotor so in Axialrichtung expandierbar ist, dass der Abstand zwischen dem ersten und dem zweiten Ende der Welle sich vergrößert. In diesem Fall ist keine axiale Beweglichkeit von feststehenden Lagerteilen des ersten oder zweiten Lagers notwendig, da die jeweils bewegten/rotierenden Lagerteile/Lagerelemente in Axialrichtung bewegbar sind. Dies kann beispielsweise dadurch realisiert werden, dass der Rotor elastisch in Axialrichtung expandierbar ist und dass ein Federelement oder eine Magnetanordnung mit wenigstens zwei Magnetelementen am Rotor vorgesehen ist, durch das oder die das erste und das zweite Ende der Welle axial auseinandergedrückt werden.

Beispielsweise kann vorgesehen sein, dass ein Längenabschnitt der Welle aus einem elastischen Werkstoff besteht, der zwischen dem ersten und dem zweiten Lager komprimiert ist. Bei einem Materialschwund durch Abrieb kann dann die Welle axial expandieren, so dass die mechanischen Führungsverhältnisse in dem ersten und zweiten Lager unverändert bleiben.

Um eine biegesteife Ausgestaltung der Welle auch bei einer Längsexpandierfähigkeit sicherzustellen, kann beispielsweise vorgesehen sein, dass die Welle zwei teleskopisch auseinanderziehbare, ineinander geführte Elemente aufweist, zwischen denen ein Federelement vorgesehen ist. Das Federelement kann beispielsweise als druckbelastete Schraubenfeder oder als Elastomerschicht ausgebildet sein.

Der Rotor kann aus zwei teleskopisch beweglichen Teilen aufgebaut sein, die kraftfrei expandierbar und z. B. durch eine kraftfreie Membran gegen Fluideintritt gesichert sind. Die Lageranpassung wird dann durch hydrodynamische Zugkraft im Betrieb auf den stromaufwärts gelegenen Teil und stromabwärts durch die magnetische Kupplung realisiert. Da der notwendige Ausgleich nur wenige Mikrometer über das Pumpenleben entspricht, kann der Teleskoprotor zu Beginn des Betriebs vollständig zusammengeschoben werden, da er sich dann im Laufe der Zeit selbstständig an die geometrischen Verhältnisse anpassen wird.

In einer weiteren Ausführungsform der Erfindung kann vorgesehen sein, dass die Blutpumpe eine Axialpumpe ist. Diese fördert unmittelbar innerhalb eines Pumpenrohrs, das den Rotor umgibt, in Axialrichtung des Pumpenrohrs oder der Welle Blut. Stromabwärts des Rotors kann das Pumpenrohr in einen erweiterten Ausströmraum führen, in dem das Blut zu einem radial außen gelegenen Pumpenauslass geleitet wird. Eine solche Axialpumpe kann jedoch auch das Blut unmittelbar in Axialrichtung in eine Kanüle oder ein sich anschließendes Rohr fördern. Die Pumpe kann jedoch auch als Halbaxialpumpe oder Radialpumpe ausgebildet sein.

Das erste und/oder das zweite Lager, in dem die Rotorwelle gelagert ist, kann jeweils als Gleitlager ausgebildet sein. Als Schmiermittel für ein derartiges Lager kann wenigstens teilweise auch das geförderte Blut dienen.

Vorteilhaft kann bei einer Blutpumpe der erläuterten Art vorgesehen sein, dass eine oder mehrere Lagerkomponenten des ersten und/oder zweiten Lagers axial verschiebbar sowie radial geführt mit einer oder mehreren in Axialrichtung des Rotors auslenkbaren und radial führenden Membranen verbunden sind.

Eine weitere Ausführungsform besteht darin, das erste Lager mit einer Axialkraft entgegengesetzt zur Förderwirkung des Rotors zu versehen und die mit dem Gehäuse verbundene Lagerkomponente axial verschiebbar zu gestalten. Die Kraft wird so eingestellt, dass sie größer als die durch den Rotor erzeugte Fluidkraft ist. Dann wird der axiale Spalt im hinteren Lager minimiert, indem der gesamte Rotor entgegen der Förderwirkung ins hintere Lager gedrückt wird. Da die beiden Kräfte (Vorspannung und Förderwirkung) in entgegengesetzte Richtungen wirken, kann die Belastung auf das zweite Lager begrenzt werden, ohne dass sich die Kräfteverhältnisse am ersten Lager während des Betriebs ändern. Diese Vorspannung kann mit einem elastischen Element oder zwei oder mehr entgegengesetzt polarisierten Magneten erzeugt werden. Lediglich bei kurzeitigen Störungen, bei der die Axialkräfte die Vorspannungskraft übersteigen (wie z. B. Ansaugung der Herzwand durch die Förderwirkung der Pumpe oder Sturz des Patienten), wird der hintere Lagerspalt kurzzeitig geöffnet. Die dann auftretende Hämolyse ist aufgrund der kurzen Zeitspanne vernachlässigbar.

Im Folgenden wird die Erfindung anhand von Figuren einer Zeichnung in Ausführungsbeispielen gezeigt und nachfolgenden erläutert. Dabei zeigt
- Fig. 1: in einem Längsschnitt eine Blutpumpe mit einer Rotorwelle, auf die in einem zweiten Lager eine magnetische Kompressionskraft wirkt,
- Fig. 2: eine Blutpumpe wie in Figur 1, wobei ein mit dem Pumpengehäuse verbundenes Lagerelement eines Lagers axial verschiebbar ist,
- Fig. 3: eine Blutpumpe ähnlich wie in Figur 1, wobei ein Federelement zur axialen Verschiebung eines mit dem Gehäuse verbundenem Lagerteil vorgesehen ist,
- Fig. 4: eine Blutpumpe, bei der eine Abdichtungsmembran für ein verschiebbares Lagerteil vorgesehen ist,
- Fig. 5: eine Blutpumpe mit einer Rotorwelle, die axial expandierbar ist,
- Fig. 6: eine Blutpumpe ähnlich wie in Figur 5, wobei die Rotorwelle zwei ineinander axial verschiebbar geführte Teile aufweist,
- Fig. 7: eine alternative Ausführungsform mit anderer Positionierung der Magnete,
- Fig. 8: eine weitere alternative Ausführungsform mit anderer Positionierung der Magnete sowie
- Fig. 9: eine Übersichtsskizze mit beiden Lagern.

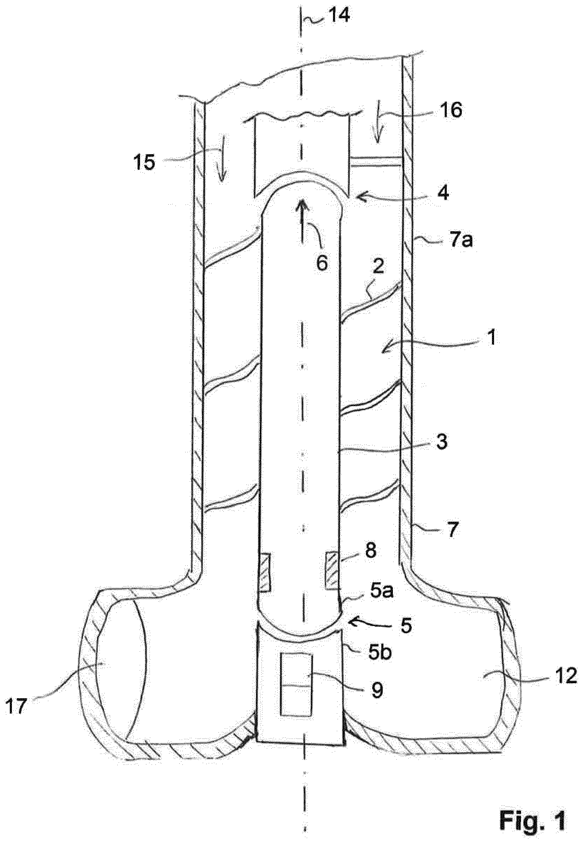

Figur 1 zeigt in einem Längsschnitt eine Blutpumpe mit einem in einem Pumpengehäuse 7 angeordneten Rotor 1. Der Rotor 1 weist eine Welle 3 auf, die Förderelemente 2 in Form von Schaufelblättern trägt. Bei einer Rotation des Rotors um die Längsachse 14 fördert der Rotor Blut in Richtung der Pfeile 15, 16 durch das Pumpenrohr 7a des Pumpengehäuses 7. Auf den Rotor wirkt durch die Rückwirkung des Blutes die axialgerichtete Kraft in Richtung des Pfeils 6, die den Rotor, genauer die Welle 3, axial in das erste Lager 4 drückt. Allgemein gelten diese Ausführungen auch für einen Rotor ohne eine dezidierte Welle, bei der der Rotorkörper in den entsprechenden, hier beschriebenen Lagern gelagert ist.

Das erste Lager 4 ist üblicherweise ein Axiallager mit einer Zentrierungsfunktion, d. h., es wirkt auch als Radiallager. Das erste Lager 4 ist typischerweise als Gleitlager ausgebildet. Es kann beispielsweise auch als hydrodynamisches Lager ausgebildet sein.

Das Blut wird durch die Pumpe entlang des Pumpenrohrs 7a bis zu dem Raum 12 der Pumpe gefördert, in dem sich das Pumpengehäuse 7 radial erweitert und aus dem das Blut teilweise tangential und radial durch einen Pumpenauslass 17 ausströmt. Alternativ kann die Pumpe auch als reine Axialpumpe ausgebildet sein, bei der der Pumpenauslass das Blut in axialer Richtung ausströmen lässt.

Die Welle 3 ist außer an dem ersten Lager 4 noch an einem zweiten Lager 5 drehbar gelagert, wobei auch das zweite Lager 5 im gezeigten Beispiel in axialer und radialer Richtung stützt.

Da die Welle 3 während des Betriebs der Pumpe durch die dynamische Rückwirkungskraft des Blutes stets in Richtung des Pfeils 6 gedrückt wird, verdient insbesondere das Lagerspiel im zweiten Lager 5 Aufmerksamkeit, das im Zuge der Abnutzung und des Schwunds an Material in beiden Lagern zunehmen kann. Wenn der Abstand zwischen dem rotierenden Element 5a und dem mit dem Pumpengehäuse 7 verbundenen Element 5b des zweiten Lagers 5 zunimmt, kann die Welle im Lager 5 schlagen, wodurch mechanische Schädigungen des Blutes zunehmen können.

Um das mechanische Spiel im zweiten Lager 5 zu verringern, ist im Bereich des mit dem Pumpengehäuse 7 verbundenen Lagerelements 5b ein Stabmagnet 9 vorgesehen, der eine Anziehungskraft auf den Ringmagnet 8 in der Welle 3 ausübt. Der Ringmagnet 8 kann vollständig in die Welle 3 oder ein mit dieser verbundenes Lagerelement eingelassen sein, so dass die Mantelfläche der Welle keine Unebenheiten zeigt.

Der Magnet kann auch allgemein im Rotorkörper vorgesehen sein. Die erzeugte Magnetkraft ist kleiner als die hydrodynamische Kraft, die den Rotor in das erste Lager 4 drückt, damit dort stets eine Andruckkraft garantiert ist. Durch die magnetische Anziehungskraft kann das Spiel im zweiten Lager 5 verringert werden, indem entweder der mit dem Pumpengehäuse verbundene Lagerteil 5b in Richtung auf den Rotor zu ausgelenkt oder im Fall einer axial expandierbaren Welle oder eines axial expandierbaren Rotors diese/dieser expandiert wird.

Alternativ kann auch in die Welle 3 ein Stabmagnet eingelassen sein, wobei im Bereich des feststehenden Lagerelements 5b des zweiten Lagers entweder ebenfalls ein Stabmagnet integriert sein kann, wie in der Figur 1 gezeigt, oder auch ein Ringmagnet ähnlich der Darstellung der Figur 1 im Bereich der Welle.

Durch die wirkenden magnetischen Kräfte zwischen dem nicht rotierenden, mit dem Pumpengehäuse verbundenen Teil/Element 5b des zweiten Lagers und dem rotierenden Element 5a wird das Spiel zwischen diesen beiden Lagerteilen tendenziell verringert. Dies geschieht einerseits durch die mögliche elastische Verformung des Pumpengehäuses oder eine axiale Verschiebung des mit dem Pumpengehäuse 7 verbundenen Lagerelements 5b in Richtung des Pfeils 6 relativ zum Pumpengehäuse, wobei der Kraft, die durch die Rückwirkung des Blutes auf die Welle 3 in Richtung des Pfeils 6 entsteht, durch die Magnetwirkung in möglichst geringem Maß entgegengewirkt wird.

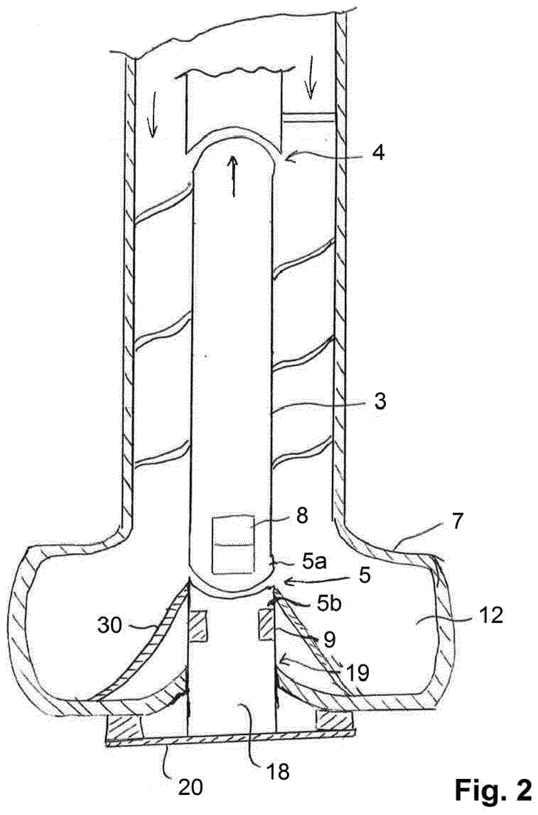

In Figur 2 ist im Längsschnitt eine Blutpumpe gezeigt, die in weiten Teilen der in der Figur 1 gezeigten Blutpumpe ähnlich ist. Im Bereich des zweiten Lagers 5 ist in die Welle 3 ein magnetisches oder magnetisierbares Element, bevorzugt ein Stabmagnet 8, integriert, während im Bereich des mit dem Pumpengehäuse verbundenen Elements 5b des Lagers ein zweites, magnetisches oder magnetisierbares Element, insbesondere in Form eines Ringmagneten, wie in der Figur gezeigt, angeordnet ist. Wenigstens eines der beiden Elemente 8, 9 ist permanentmagnetisch, damit eine magnetische Anziehungskraft erzeugt wird. Der zylindrische Körper 18, der den mit dem Pumpengehäuse 7 verbundenen Teil des zweiten Lagers 5b trägt, ist an dem Pumpengehäuse 7 geringfügig in Axialrichtung verschiebbar angeordnet. Um den Spalt zwischen dem Körper 18 und dem Pumpengehäuse 7, der in der Figur mit 19 bezeichnet ist, abzudichten, ist an der Außenwand des Pumpengehäuses eine zusätzliche Membran 20 vorgesehen, die mit dem Pumpengehäuse 7 fluiddicht verbunden ist. Dadurch kann Blut, das aus dem Pumpenraum 12 durch den Spalt 19 austritt, nicht zum Pumpenäußeren gelangen.

Alternativ oder zusätzlich kann der Spalt 19 zwischen dem Körper 18 und dem Pumpengehäuse 7 auch durch eine passende Gleitdichtung oder einen Faltenbalg oder eine faltenbalgähnliche Anordnung oder Membran 30 abgedichtet sein. Bei der axial beweglichen Halterung des Lagerteils 5b sollte zusätzlich ein Verdrehschutz vorgesehen sein.

Dadurch, dass der zylindrische Körper 18 in Axialrichtung bewegbar ist, kann durch die magnetische Anziehungskraft zwischen den Magneten 8, 9 das Spiel im Lager 5 minimiert werden, ohne dass eine wesentliche Änderung der Kräfte im Lager 4 stattfindet.

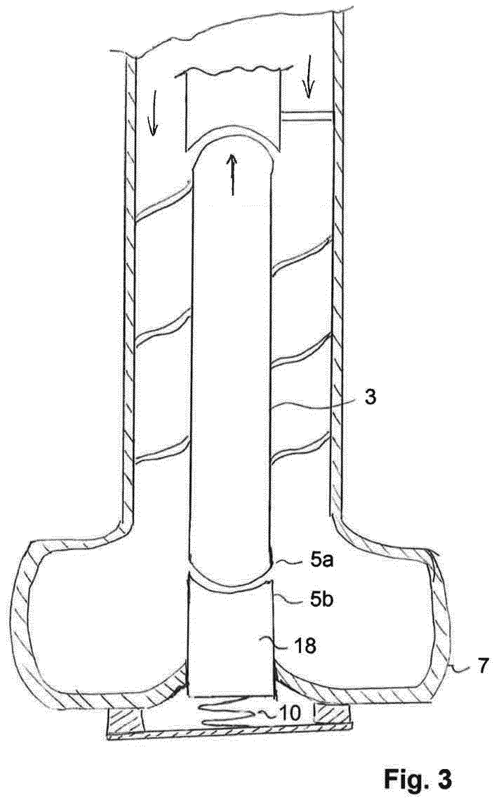

Figur 3 zeigt eine Konstruktion, die der in Figur 2 dargestellten ähnlich ist, wobei der zylindrische Körper 18 gegenüber dem Pumpengehäuse 7 wieder axial verschiebbar ist, wobei jedoch anstelle der in Figur 2 dargestellten Magnete eine Feder 10 vorgesehen ist, die den Körper 18 und damit das mit dem Pumpengehäuse 7 verbundene Elemente 5b des Lagers 5 in Richtung der Welle 3 mit einer Kraft belastet. Dadurch wird eine Kompressionskraft zwischen dem beweglichen und dem feststehenden Teil des Lagers 5 erzeugt, um das Spiel in diesem Lager dauerhaft zu minimieren.

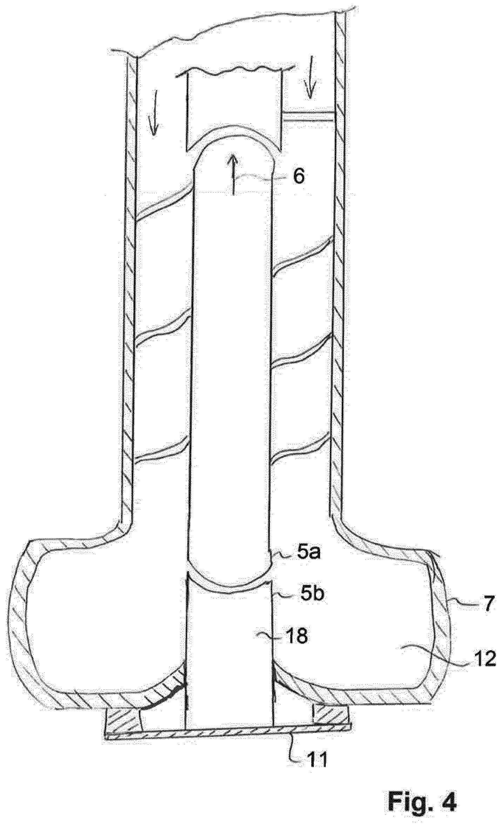

In Figur 4 ist eine Konstruktion dargestellt, die der Konstruktion aus Figur 3 ähnelt, wobei jedoch die Feder 10 weggelassen ist und ihre elastische Funktion durch die Membran 11 übernommen wird. Diese kann beispielsweise aus einem dünnen Federblech oder einem Elastomer bestehen und eine federnde Wirkung auf den Körper 18 in Richtung des Pfeils 6 zur axialen Komprimierung der Lagerelemente des Lagers 5 ausüben.

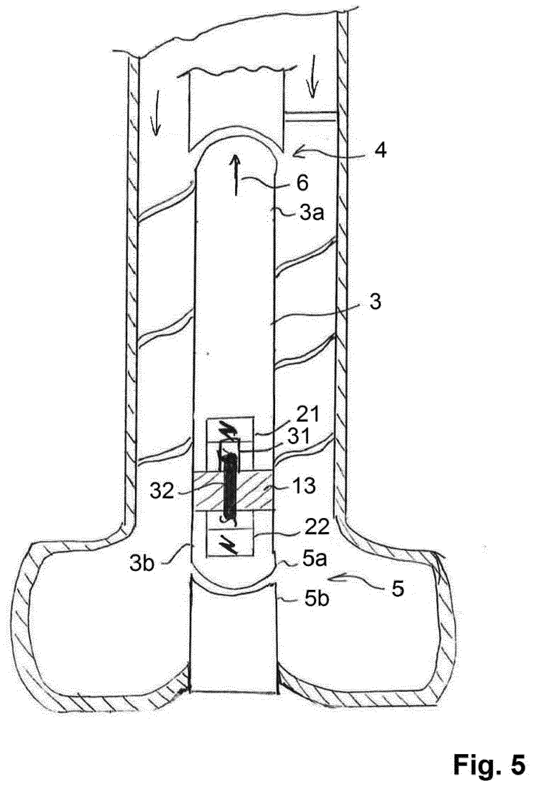

Figur 5 zeigt eine Variante der Erfindung, bei der die Welle 3 selbst in axialer Richtung 6 expandierbar ist. Die Welle 3 ist geteilt in einen dem ersten Ende 3a zugewandten Teil und einen dem zweiten Ende 3b zugewandten Teil, die durch einen elastischen Längsabschnitt 13 miteinander verbunden sind. Dieser elastische Längsabschnitt 13 kann beispielsweise aus einem relativ steifen Elastomer bestehen und in axialer Richtung vorgespannt sein, so dass er dauerhaft auf die Welle 3 zwischen dem ersten Lager 4 und dem zweiten Lager 5 eine Expansionskraft in axialer Richtung ausübt. Es kann jedoch auch vorgesehen sein, dass der Längsabschnitt 13 lediglich flexibel ist und dass auf beiden Seiten des Längsabschnitts 13 jeweils ein Magnet 21, 22 vorgesehen ist, wobei die beiden Magnete derart angeordnet sind, dass sie sich gegenseitig abstoßen. Damit ist dauerhaft eine Expansionskraft in axialer Richtung innerhalb der Welle 3 selbst gegeben, die die Wellenenden 3a, 3b in die Lager 4, 5 drückt. Vorteilhaft kann eine den Längsabschnitt 13 in Axialrichtung überbrückende Axialführung, beispielsweise in Form eines Stabes 32, vorgesehen sein, der auf wenigstens einer Seite des Längsabschnitts 13 axial beweglich in einer Ausnehmung 31 spielfrei geführt ist und auf der anderen Seite des Längsabschnitts 31 entweder ebenfalls spielfrei geführt oder festgehalten ist.

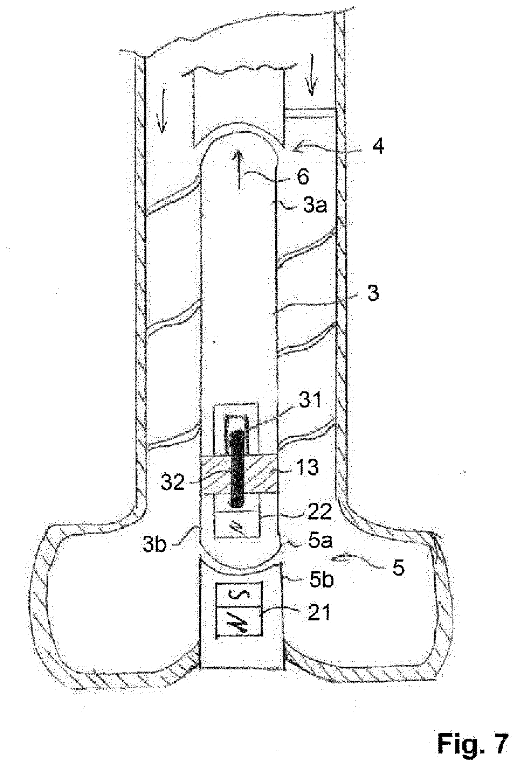

Der Magnet 21 kann alternativ, wie in Figur 7 gezeigt, auch in dem Körper 18 angeordnet sein, wobei dann die Magnete 21, 22 derart angeordnet sind, dass sie sich gegenseitig anziehen.

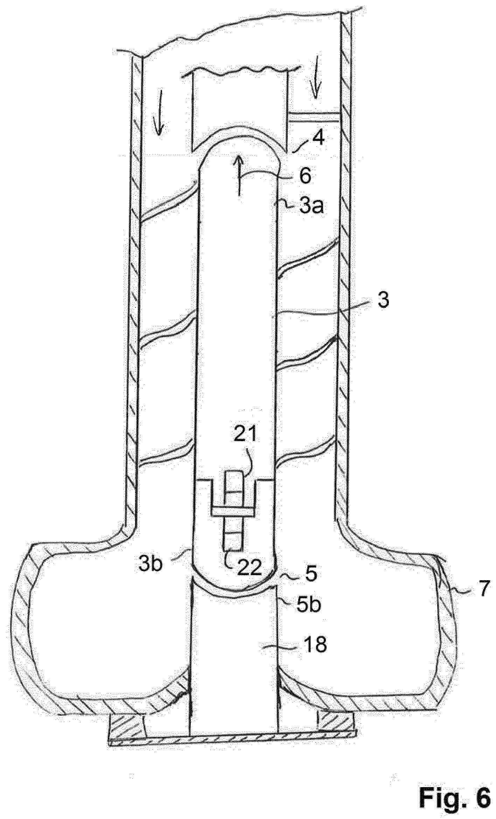

Die Figur 6 zeigt in Abänderung der Konstruktion aus der Figur 5 ebenfalls eine Blutpumpe mit einer in Axialrichtung 6 expandierbaren Welle 3, wobei der erste, dem ersten Ende 3a der Welle zugewandte Teil der Welle innerhalb einer Ausnehmung des zweiten Teils der Welle, welche dem zweiten Ende 3b zugewandt ist, in Axialrichtung gleitend geführt ist. Dadurch ist eine teleskopartige Konstruktion der Welle 3 gegeben, die eine gute Biegesteifigkeit der Welle garantiert und dabei dennoch eine axiale Expandierbarkeit ermöglicht. Einer oder beide Teile der Welle können auch jeweils an einem dritten Teil axial verschiebbar geführt sein, das den Zwischenraum zwischen den beiden Teilen überbrückt und stab- oder rohrförmig ausgebildet ist. Die beiden Teile können mit jeweils einem Magneten 21, 22 bestückt sein, wobei die beiden Magnete sich gegenseitig abstoßen und damit eine expandierende Axialkraft auf die Teile der Welle ausüben. Damit wird eine Andruckkraft in den beiden Lagern 4, 5 erzeugt. Der mit dem Pumpengehäuse 7 verbundene Teil 5b des Lagers 5, der an dem zylindrischen Körper 18 angeordnet ist, kann in diesem Fall in Axialrichtung 6 verschiebbar und gegebenenfalls federbelastet oder auch fest und unbeweglich mit dem Pumpengehäuse 7 verbunden sein.

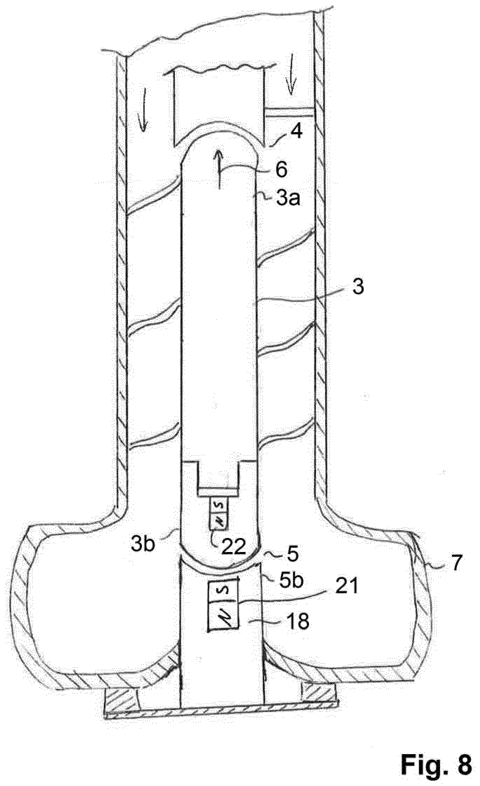

Eine weitere, der in Figur 6 dargestellten Ausführungsform ähnliche Variante ist in Figur 8 gezeigt. Der Unterschied der in Figur 8 gezeigten Version zu der in Figur 6 gezeigten Ausführungsform besteht darin, dass der Magnet 22 im Rotor und der Magnet 21 in dem mit dem Pumpengehäuse verbundenen Körper 18 angeordnet ist. Die beiden Magnete 21, 22 ziehen sich dann gegenseitig an, so dass die Welle expandiert wird, wobei die Kräfte im ersten Lager 4 im Wesentlichen unbeeinflusst bleiben. Die Form der Magnete 21, 22 ist nicht auf Stabmagnete beschränkt, sondern jegliche sinnvolle Form von Magneten ist einsetzbar.

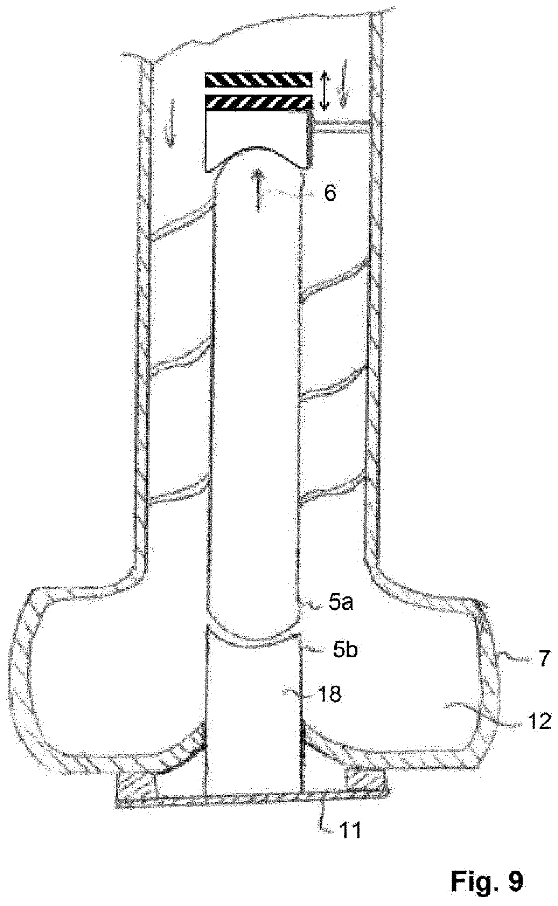

Figur 9 zeigt in einer Übersichtsskizze den Rotor mit dem ersten Lager 4 und dem zweiten Lager 5. Im Bereich des ersten Lagers 4 ist schematisch ein Ausgleichsmechanismus dargestellt, der den mit dem Pumpengehäuse verbundenen Lagerteil derart in Richtung des Rotors nachführt, dass beide axialen Lagerspalte, insbesondere aber der Axialspalt des zweiten Lagers 5, begrenzt werden.

Die gezeigten und erläuterten Konstruktionen erlauben durch die Minimierung des Spiels im zweiten Lager der Welle 3 jeweils einen dauerhaften Einsatz der beschriebenen Blutpumpe unter Minimierung hämolytischer Effekte.

Im Zusammenhang mit der Erfindung kann zudem vorgesehen sein, dass
- die Kompression zwischen dem Pumpengehäuse und dem ersten Lager erzeugt wird,
- die Kompression durch ein elastisches Element erzeugt wird und
- die Vorspannung/Kompression durch zwei Magnete erzeugt wird.

Zudem kann sich die Erfindung beziehen auf ein Verfahren zur Kontrolle der axialen Lagerspalte in einer mechanisch gelagerten Pumpe, die auf der Nutzung einer einseitigen Kompressionsvorrichtung im mechanischen Lager beruht, derart dass die Lagerspalte definiert zugefahren werden, ohne dass eine wesentliche zusätzliche Kraft auf das zweite mechanischen Lager aufgebaut wird.

## Patentansprüche

1. Blutpumpe mit einem antreibbaren Rotor (1), der ein oder mehrere Förderelemente (2) zur Förderung von Blut aufweist und der an seinem ersten Ende (3a) in einem ersten Lager (4) und an seinem zweiten Ende (3b) in einem zweiten Lager (5) drehbar gelagert ist, wobei die Form und Rotationsrichtung des Rotors derart eingerichtet sind, dass im Betrieb eine Rückwirkungskraft des geförderten Blutes den Rotor in Axialrichtung (6) gegen das erste Lager (4) drückt,
**dadurch gekennzeichnet, dass** eine Kompressionseinrichtung (8, 9, 10, 11, 21, 22) vorgesehen ist, die eine axiale Kompressionskraft zwischen einem mit dem Rotor (1) rotierenden Lagerelement (5a) eines Lagers, insbesondere des zweiten Lagers, und einem mit dem Pumpengehäuse (7) verbundenen Element (5b) des Lagers (5), insbesondere des zweiten Lagers, erzeugt.

2. Blutpumpe nach Anspruch 1, **dadurch gekennzeichnet, dass** die Kompressionseinrichtung (8, 9, 10, 11, 21, 22) wenigstens zwei Magnetelemente in Form zweier Magnete oder eines Magneten und eines magnetisierbaren Elements aufweist.

3. Blutpumpe nach Anspruch 1 oder 2, **dadurch gekennzeichnet, dass** eines oder beide Lagerelemente (5a, 5b) des zweiten Lagers relativ zu dem Pumpengehäuse und/oder dem ersten Ende des Rotors axial verschiebbar sind.

4. Blutpumpe nach Anspruch 1, 2 oder 3, **dadurch gekennzeichnet, dass** die Kompressionseinrichtung ein erstes Magnetelement, insbesondere einen ersten Magneten (8), aufweist, der mit dem Rotor (1) verbunden ist, sowie ein zweites Magnetelement, insbesondere einen zweiten Magneten (9), der mit dem Pumpengehäuse (7) verbunden und axial vor oder hinter dem zweiten Ende (3b) des Rotors (1) angeordnet ist, wobei zwischen dem ersten und dem zweiten Magneten eine Anziehungskraft oder Abstoßungskraft wirkt.

5. Blutpumpe nach Anspruch 1, 2, 3 oder 4, **dadurch gekennzeichnet, dass** ein mit dem Pumpengehäuse (7) verbundenes Element (5b) des zweiten Lagers (5) am Pumpengehäuse (7) oder ein mit dem Rotor verbundenes Element (5a) des zweiten Lagers relativ zu dem ersten Ende des Rotors axial verschiebbar ist.

6. Blutpumpe nach Anspruch 1, 2, 3, 4 oder 5, **dadurch gekennzeichnet, dass** ein mit dem Pumpengehäuse (7) verbundenes Element (5b) des ersten oder zweiten Lagers (5) am Pumpengehäuse (7) mittels eines Federelements (10, 11) axial verschiebbar abgestützt ist, wobei das Federelement derart vorgespannt ist, dass es das mit dem Pumpengehäuse (7) verbundene Lagerelement (5b) in Axialrichtung gegen das rotierende Lagerelement (5a) des ersten oder zweiten Lagers drückt.

7. Blutpumpe nach Anspruch 1, 2, 3, 4, 5 oder 6, **dadurch gekennzeichnet, dass** das axial verschiebbare, mit dem Pumpengehäuse (7) verbundene Lagerelement (5b) des zweiten Lagers (5) wenigstens teilweise in dem blutdurchströmten Raum (12) der Pumpe angeordnet und gegenüber dem Außenraum durch eine fluiddichte Membran (11) abgedichtet ist.

8. Blutpumpe nach Anspruch 7, **dadurch gekennzeichnet, dass** die Membran (11) elastisch ausgebildet ist und das Federelement bildet, das das mit dem Pumpengehäuse (7) verbundene Lagerelement (5b) des zweiten Lagers gegen das rotierende Lagerelement (5a) des zweiten Lagers drückt.

9. Blutpumpe nach einem der Ansprüche 1 bis 8, **dadurch gekennzeichnet, dass** der Rotor (1) derart in Axialrichtung expandierbar ist, dass sich der Abstand zwischen dem ersten und dem zweiten Ende (3a, 3b) der Welle (3) vergrößert.

10. Blutpumpe nach Anspruch 9, **dadurch gekennzeichnet, dass** der Rotor (1) elastisch in Axialrichtung expandierbar ist und dass ein Federelement (13) oder eine Magnetanordnung mit wenigstens zwei Magneten am Rotor vorgesehen ist, durch das oder die das erste und das zweite Ende (3a, 3b) der Welle (3) axial auseinandergedrückt werden.

11. Blutpumpe nach einem der Ansprüche 1 bis 10, **dadurch gekennzeichnet, dass** die Blutpumpe eine Axialpumpe oder eine halbaxiale Pumpe oder eine Radialpumpe ist.

12. Blutpumpe nach einem der Ansprüche 1 bis 11, **dadurch gekennzeichnet, dass** das zweite Lager (5) ein Gleitlager ist.

13. Blutpumpe nach einem der Ansprüche 1 bis 12, **dadurch gekennzeichnet, dass** das erste Lager (4) ein Gleitlager ist.

14. Blutpumpe nach einem der Ansprüche 1 bis 13, **dadurch gekennzeichnet, dass** eine oder mehrere Lagerkomponenten des ersten und/oder zweiten Lagers axial verschiebbar sowie radial geführt mit einer oder mehreren in Axialrichtung des Rotors auslenkbaren und radial führenden Membranen verbunden sind.

15. Blutpumpe nach Anspruch 1, bei dem die Kompression zwischen dem Pumpengehäuse und dem ersten Lager erzeugt wird.

16. Lagerkonfiguration nach Anspruch 15, bei dem die Kompression durch ein elastisches Element erzeugt wird.

17. Lagerkonfiguration nach Anspruch 15, bei dem die Vorspannung durch zwei Magnete erzeugt wird.

18. Verfahren zur Kontrolle der axialen Lagerspalte in einer mechanisch gelagerten Pumpe, die auf der Nutzung einer einseitigen Kompressionsvorrichtung im mechanischen Lager beruht, derart dass die Lagerspalte definiert zugefahren werden, ohne dass eine wesentliche zusätzliche Kraft auf das zweite mechanische Lager aufgebaut wird.
